# EUROPÄISCHE PATENTANMELDUNG

(11) **EP 2 301 660 A1**
(43) Veröffentlichungstag der Anmeldung: **30.03.2011**
(21) Anmeldenummer: 09171391.7
(22) Anmeldetag: 25.09.2009
(51) Int. Cl.: B01J 23/42, B01J 23/89, C07C 209/36

(54) **Verfahren zur Hydrierung von halogenierten Nitroaromaten**

(71) Anmelder: LANXESS Deutschland GmbH, 51369 Leverkusen (DE)
(72) Erfinder: Menzel, Thomas, 42799, Leichlingen (DE); Boll, Matthias, 51061, Köln (DE)

(57) **Zusammenfassung**

Die Erfindung betrifft ein Verfahren zur Hydrierung von halogenierten Nitroaromaten zu den entsprechenden Aminovcrbindungcn unter Verwendung halogcnidhaltigcr Katalysatoren.

## Beschreibung

Die Erfindung betrifft ein Verfahren zur Hydrierung von halogenierten Nitroaromaten zu den entsprechenden Aminoverbindungen unter Verwendung halogenidhaltiger Katalysatoren.

Die Hydrierung von Nitroaromaten zu den entsprechenden Aminen wird in der chemischen Industrie weltweit großtechnisch durchgeführt. Grundsätzlich sind für solche Hydrierungen eine ganze Reihe von Katalysatoren geeignet, zum Beispiel Nickel- oder Kobaltkatalysatoren in Form der entsprechenden Raney-Legierungen oder auch fein verteilt auf Trägermaterialien.

Neben den vorgenannten Katalysatortypen werden häufig auch Edelmetallkatalysatoren auf Trägersubstanzen eingesetzt, wie zum Beispiel Platin oder Palladium auf Aktivkohle.

Der Selektivität der Hydrierung kommt generell eine besondere Bedeutung zu, weil bereits geringe Verbesserungen der Selektivität aufgrund ihres positiven Einflusses auf die Eduktkosten und des geringeren Nebenproduktanfalls zu großen wirtschaftlichen Vorteilen führen.

Insbesondere wird bei der Hydrierung von halogenierten Nitroaromaten als Konkurrenzreaktion Dehalogenierung beobachtet, deren weitestgehende Vermeidung hohe Anforderungen an das Katalysatorsystem stellt.

Es ist beispielsweise aus CN 1861253 A bekannt, zur Gasphasenhydrierung von o-Nitrotoluol Kupferkatalysatoren auf Silica einzusetzen, die weiterhin Nickel bzw. Palladium enthalten können. Weiterhin ist für den gleichen Zweck der Einsatz von Kobalt und Nickel enthaltenden, geträgerten Platinkatalysatoren aus DE 39 28 329 OS bekannt.

Darüber hinaus beschreibt CN 101049560 A zur Hydrierung von Dichlomitrobenzolen Palladiumkatalysatoren auf chemisch aktivierten Aktivkohlen, wobei speziell für die Hydrierung von 3,4-Dichlornitrobenzol in CN 1817455 A mit Alkalihalogeniden versetzte, palladiumhaltige Katalysator auf Aktivkohle als Trägermaterial vorgeschlagen werden.

Allen vorgenannten Katalysatorsystemen ist jedoch gemein, dass sie entweder ungenügende Umsatzgeschwindigkeiten oder verminderte Selektivitäten bei erhöhten Temperaturen aufweisen.

Aufgabe der vorliegenden Erfindung war es daher, ein Verfahren zur Herstellung von halogenierten Aminoaromaten durch Hydrierung der entsprechenden halogenierten

Nitroaromaten zur Verfügung zu stellen, das die Nachteile des Standes der Technik überwindet.

Gegenstand der Erfindung ist nun ein Verfahren zur Herstellung von Verbindungen der Formel (I) 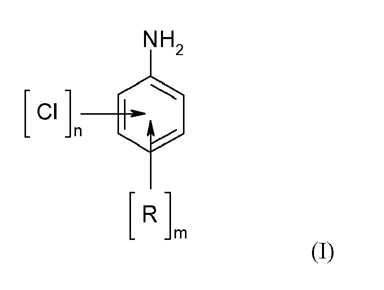 in der
n für 1, 2 oder 3 steht und
m für 0, 1, 2 oder 3 steht
wobei die Summe aus m + n = 1, 2, 3, 4 oder 5 ist und
R für C₁-C₆-Alkyl steht durch Hydrierung von Verbindungen der Formel (II) 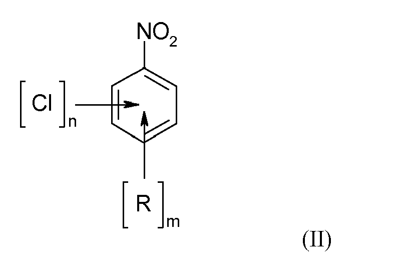 in der n, m und R die unter der Formel (I) genannte Bedeutung besitzen, in Gegenwart zumindest eines Katalysators, das dadurch gekennzeichnet ist, dass der Katalysator zumindest enthält:
a) Platin und
b) Bromid oder Iodid oder Bromid und Iodid
c) Ionen zumindest eines Alkali- oder Erdalkalimetalls.

Es sei an dieser Stelle angemerkt, dass der Rahmen der Erfindung alle beliebigen und möglichen Kombinationen der oben stehenden und im Folgenden aufgeführten, allgemeinen oder in Vorzugsbereichen genannten Komponenten, Wertebereiche bzw. Verfahrensparameter umfasst.

Bevorzugt werden nach dem erfindungsgemäßen Verfahren solche Verbindungen der Formel (II) verwendet bzw. solche der Formel (I) hergestellt, in denen n = 2 und m = 1 oder 0, bevorzugt 0 ist. Besonders bevorzugt werden 2,3-Dichloranilin, 2,5-Dichloranilin, 3,4-Dichloranilin, 2,4-Dichloranilin, 3,5-Dichloranilin, 2,6-Dichloranilin oder Mischungen davon hergestellt, wobei 3,4-Dichloranilin bevorzugt ist.

Bevorzugte Katalysatoren sind solche, die zumindest Platin und Iodid sowie Ionen zumindest eines Alkali- oder Erdalkalimetalls enthalten.

In einer weiteren Ausführungsform werden Katalysatoren eingesetzt, die als Übergangsmetall ausschließlich Platin enthalten oder solche, die als Übergangsmetalle Platin und zumindest ein weiteres Übergangsmetall enthalten, das ausgewählt ist aus der Gruppe Nickel und Kobalt, wobei solche Katalysatoren bevorzugt sind, die als Übergangsmetalle Platin und Nickel oder Platin und Kobalt enthalten, wobei solche Katalysatoren besonders bevorzugt sind, die als Übergangsmetalle Platin und Nickel enthalten.

Weiterhin sind Katalysatoren bevorzugt, in denen Platin sowie die gegebenenfalls weiterhin vorhandenen Übergangsmetalle auf ein Trägermaterial aufgebracht sind. Geeignete Trägermaterialien sind beispielsweise anorganische Trägermaterialien wie vorzugsweise Aktivkohle, Aluminiumoxid, Aluminiumsilicat und andere Silikate, anorganische Keramiken, Siliciumdioxid, Bariumsulfat, Calciumcarbonat, Cerdioxid, Titandioxid und Zirkondioxid. Bevorzugte Trägermaterialen sind Aktivkohle und Aluminiumoxid, wobei Aktivkohle noch weiter bevorzugt ist.

Als Aktivkohlen können prinzipiell alle bekannten eingesetzt werden. Bevorzugte Aktivkohlen sind solche, die aus Holzkohle, Braunkohle, Steinkohle, Torf, Holz, Kokosnussschalen oder Olivenkernen hergestellt werden. Hinsichtlich der Eigenschaften, wie z.B. der BET-Oberfläche der Trägermaterialien, liegen prinzipiell keine Einschränkungen vor. Gleiches gilt für die Haptik der Trägermaterialen, wobei jedoch pulverformige Trägermaterialien bevorzugt sind. Vorzugsweise beträgt der Gehalt an Platin bei Katalysatoren, die auf ein Trägermaterial aufgebracht sind 0,1 bis 20 Gew.-%, bevorzugt 0,5 bis 10 Gew.-% und besonders bevorzugt 0,5 bis 2 Gew.-%, bezogen auf den Katalysator in der reduzierten und trockenen Form.

Der Begriff "in der reduzierten Form" bedeutet dabei, dass die Angaben zu den Gehalten auf die Metalle selbst berechnet werden, selbst wenn sie teilweise oder vollständig in Form von Metallsalzen vorliegen. Der Begriff "trockene Form" bedeutet dabei, dass die Angaben zu den Metallgehalten auf den trockenen Katalysator, d.h. ohne anhaftendes Wasser und/oder Lösungsmittel bezogen sind.

Vorzugsweise beträgt der Gehalt an weiteren Übergangsmetallen, sofern sie vorhanden sind, 0,1 bis 5 Gew.-%, bevorzugt 0,2 bis 1 Gew.-%, bezogen auf den Katalysator in der reduzierten und trockenen Form.

Vorzugsweise beträgt in solchen Katalysatoren das Gewichtsverhältnis von Platin und weiteren Übergangsmetallen zwischen 1:1 und 10:1 bevorzugt zwischen 3:1 und 10:1.

Besonders bevorzugt sind Katalysatoren auf Aktivkohle, die Platin und Nickel enthalten, besonders bevorzugt sind solche die zwischen 0,1 und 10 Gew.-%, bevorzugt zwischen 0,5 und 2 Gew.-% Platin und zwischen 0,01 und 5 Gew.-% bevorzugt 0,1 bis 0,3 Gew.-% Nickel enthalten. Ein ganz besonders bevorzugter Katalysator enthält zwischen 0,5 und 2 Gew.-% Platin und 0,1 bis 0,3 Gew.-% Nickel und ist auf Aktivkohle aufgebracht.

Bromid und Iodid, bevorzugt Iodid können beispielsweise in Form von anorganischen oder organischen Bromiden oder Iodiden im Katalysator vorhanden sein oder eingebracht werden, wobei anorganischen Bromide oder Iodide bevorzugt sind.

Da weiterhin Ionen zumindest eines Alkali- oder Erdalkalimetalls im Katalysator enthalten sein müssen, sind bevorzugte anorganische Iodide und Bromide beispielsweise Lithium-, Natrium-, Kalium-, Rubidium-, Cäsium-, Calcium-, Strontium und Bariumiodid oder die Bromide der vorgenannten Alkali- und Erdalkalimetalle, wobei die entsprechenden Iodide bevorzugt, Lithium-, Natrium-, Kalium-, Rubidium- und Cäsiumiodid besonders bevorzugt und Kaliumiodid ganz besonders bevorzugt sind.

Geeignete organische Iodide und Bromide sind beispielsweise Ammoniumiodid oder Bromid oder primäre, sekundäre, tertiäre oder quaternäre organische Ammoniumiodide oder -bromide, Iodide oder Bromide von Heterocyclen mit tertiären oder quaternären Stickstoffatomen wie Pyridiniumiodide und -bromide oder Alkylimidazoliumiodide oder -bromide wobei die jeweiligen Iodide bevorzugt sind.

Vorzugsweise beträgt das molare Verhältnis von Bromid oder Iodid zur Gesamtmenge an Übergangsmetallen im Katalysator 2:1 bis 100:1 vorzugsweise 5:1 bis 50:1 und besonders bevorzugt 5:1 bis 20:1.

Bei der Verwendung von Alkalimetallionen beträgt das molare Verhältnis von Alkalimetallionen zur Gesamtmenge an Übergangsmetallen im Katalysator 2:1 bis 100:1 vorzugsweise 5:1 bis 50:1 und besonders bevorzugt 5:1 bis 20:1.

Bei der Verwendung von Erdalkalimetallionen beträgt das molare Verhältnis von Erdalkalimetallionen zur Gesamtmenge an Übergangsmetallen im Katalysator 1:1 bis 5:1 vorzugsweise 2,5:1 bis 25:1 und besonders bevorzugt 2,5:1 bis 10:1.

Vorzugsweise beträgt das molare Verhältnis von Bromid oder Iodid zur Gesamtmenge an Alkalimetallionen oder zur halben Menge an Erdalkalimetallionen im Katalysator 5:1 bis 1:5 vorzugsweise 1,5:1 bis 1:1,5 und besonders bevorzugt 1,1:1 bis 1:1,1.

Die entsprechenden nicht Iodid oder Bromid oder (Erd-)Alkalimetallionen enthaltenden Katalysatoren sind prinzipiell bekannt. Die Herstellung kann in für den Fachmann an sich bekannter Weise erfolgen.

Die Herstellung der Katalysatoren kann beispielsweise nasschemisch durch Imprägnierung in der Weise erfolgen, dass eine bestimmte Menge einer Platin-Vorstufe, wie beispielsweise Hexachloroplatinate oder Platinchlorid, sowie gegebenenfalls eine bestimmte Menge einer oder mehrerer anderer Übergangsmetallverbindungen in einem geeigneten Lösungsmittel und/oder Wasser vorgelegt werden und unter Rühren das Trägermaterial zugesetzt wird. Danach trennt man den Feststoff von der flüssigen Phase ab, beispielsweise durch Filtration, Sedimentation oder Zentrifugation. Gegebenenfalls aber bevorzugt kann anschließend eine Reduktion erfolgen. Geeignete Reduktionsmittel sind beispielsweise Wasserstoff oder Hydrazinhydrat.

Die Zugabe von Iodid oder Bromid kann dann in einem weiteren Schritt beispielsweise durch einfaches Imprägnieren der nicht Iodid oder Bromid enthaltenden Katalysatoren mit wässrigen Lösungen der vorgenannten anorganischen oder organischen Iodide bzw. Bromide erfolgen oder durch Zugabe von Iodid oder Bromid zu dem zu hydrierenden Gemisch enthaltend einen nicht Iodid oder Bromid enthaltenden Katalysator. Gleiches gilt für den Zusatz von Ionen zumindest eines Alkalimetalls oder Erdalkalimetalls.

Die für das erfindungsgemäße Verfahren einsetzbaren Katalysatoren sind bislang unbekannt. Weiterer Gegenstand der Erfindung sind daher Katalysatoren enthaltend:
a) Platin und
b) Bromid oder Iodid oder Bromid und Iodid
c) Ionen zumindest eines Alkali- oder Erdalkalimetalls.
wobei die vorgenannten für die Katalysatoren genannten Vorzugsbereiche naturgemäß in gleicher Weise gelten.

Das erfindungsgemäße Verfahren wird so durchgeführt, dass der Katalysator mit den Verbindungen der Formel (II) und Wasserstoff gegebenenfalls in Gegenwart eines Lösungsmittels in Kontakt gebracht wird.

Das erfindungsgemäße Verfahren kann entweder in Abwesenheit oder in Gegenwart von Lösungsmitteln durchgeführt werden. Lösungsmittel im Sinne der Erfindung sind dabei nicht die Edukte und Produkte der Hydrierung sondern andere Verbindungen, die mit den eingesetzten Materialien nicht in unerwünschter Weise reagieren und ein ausreichendes Lösevermögen für die Edukte und/oder Produkte haben. Beispiele sind aliphatische Kohlenwasserstoffe wie Hexan und Isooctan, gegebenenfalls halogenierte, aromatische Kohlenwasserstoffe wie Toluol, Xylole oder Chlorbenzol, lineare und cyclische aliphatische Ether wie tert.-Butyl-methyl-ether und Tetrahydrofuran, C₁-C₈-Alkyl und C₇-C₁₀-Aralkylalkohole wie Methanol, Ethanol, n-Propanol, i-Propanol und Benzylalkohol oder Mischungen der vorgenannten Lösungsmittel.

Bevorzugte Lösungsmittel sind C₁-C₄-Alkylalkohole, wobei i-Propanol besonders bevorzugt ist. Die Gegenwart von Wasser ist unschädlich. Wasser wird auch als Reaktionsprodukt erzeugt.

Das erfindungsgemäße Verfahren kann beispielsweise bei einem Wasserstoffpartialdruck von 0,1 bis 40 MPa, bevorzugt 2 bis 30 MPa und besonders bevorzugt bei 5 bis 25 MPa und ganz besonders bevorzugt bei 5 bis 20 MPa durchgeführt werden. Typischerweise entspricht der Wasserstoffpartialdruck in etwa dem Reaktionsdruck.

Die Reaktionstemperatur kann beim erfindungsgemäßen Verfahren beispielsweise im Bereich von 20 bis 250°C liegen. Vorzugsweise liegt sie in einem Bereich von 100 bis 250°C, besonders bevorzugt bei 100 bis 200°C und ganz besonders bevorzugt 130 bis 180°C.

Das erfindungsgemäße Verfahren kann grundsätzlich batchweise, z.B. als sogenannte Pumphydrierung, oder auch kontinuierlich z.B. als sogenannte Konti-Hydrierung durchgeführt werden. Eingesetzt werden können dabei handelsübliche Druckautoklaven, wie sie nach dem Stand der Technik für Hydrierungen typischerweise verwendet wurden.

Die Reaktionszeit ist abhängig von der Ausführungsform des Verfahrens und den Reaktionsbedingungen. Sie liegt im Allgemeinen in einem Bereich von beispielsweise 5 Minuten bis 24 Stunden.

In einer bevorzugten Ausführungsform wird das Verfahren kontinuierlich bei einer mittleren Verweilzeit des Edukts von 10s bis 30 min , vorzugsweise 10s bis 5 min durchgeführt. In diesem Fall liegt der Wasserstoffpartialdruck vorzugsweise bei 5 bis 20 MPa, die Temperatur bei 100 bis 200°C.

Kontinuierlich geführte Reaktionen können prinzipiell beliebig lange zum Beispiel über mehrere Tage oder Wochen durchgeführt werden.

Die Menge des in das erfindungsgemäße Verfahren eingesetzten Katalysators ist abhängig von der Ausführungsform des Verfahrens und kann bei Durchführung in Batchfahrweise beispielsweise von 1:1 bis 1:100000, bevorzugt zwischen 1:100 bis 1:10000 bezogen auf das Gewichtsverhältnis des Katalysators in der trockenen Form zum Substrat betragen.

Bei kontinuierlich geführten Reaktionen kann das Gewichtsverhältnis des Katalysators in der trockenen Form zum Substrat, das pro Stunde über den Katalysator geführt wird, beispielsweise 1:1 1 bis 1:5000, bevorzugt zwischen 1:100 und 1:1000 betragen.

Die Aufarbeitung des Reaktionsgemisches erfolgt typischerweise dadurch, dass der Katalysator beispielsweise durch Filtration, Sedimentation oder Zentrifugation, bevorzugt durch Filtration abgetrennt wird. In einer bevorzugten Ausführungsform wird der isolierte Katalysator wieder in die Reaktion zurückgeführt bzw. wiederverwendet.

Die erfindungsgemäß hergestellten Verbindungen der Formel (I) eignen sich insbesondere zur Herstellung von pharmazeutischen oder agrochemischen Wirkstoffen, wie beispielsweise Diuron. Daher ist ein weitere Gegenstand der Erfindung ein Verfahren zur Herstellung von pharmazeutischen oder agrochemischen Wirkstoffen, das zumindest das vorgenannte Verfahren zur Herstellung von Verbindungen der Formel (I) umfasst.

Der besondere Vorteil der Erfindung liegt darin, dass hohe Raum-Zeit-Ausbeuten bei gleichzeitiger sehr hoher Produktselektivität von typischerweise über 99 %realisiert werden können, womit die bisher in der Regel erforderliche Feindestillation des Rohproduktes entbehrlich ist.

Die folgenden Beispiele sollen das erfindungsgemäße Verfahren weiter erläutern, ohne es jedoch in seinem Umfang zu begrenzen.

### Beispiele:

Alle Versuche wurden vergleichend unter identischen Bedingungen mit einer Mischung bestehend aus 94,56 Gew.-3,4-Dichloranilin (3,4-DCBA) und 5,44 Gew.-% 2,3-Dichlomitrobenzol (2,3-DCBA) als Edukt durchgeführt.

Als Katalysator wurde jeweils ein kommerziell verfügbarer Pt/Ni/Aktivkohlekatalysator mit der Zusammensetzung 1,0 Gew-% Platin; 0,2 Gew-Nickel auf Aktivkohle des Typs Norit CN1, einer chemisch aktivierten neutralen Holzkohle, eingesetzt, der mit den in Tabelle 1 angebenen Mengen an Iodid versetzt wurde.

### Versuchsdurchführung:

In einem Druckautoklaven wurden jeweils 1,00 g Katalysator (angegeben als Trockengewicht) mit 187 g Isopropanol, das einen Gehalt von ca. 12 Gew.-% Wasser aufwies, vorgelegt. Dazu wurden die in Tabelle 1 angegebenen Mengen an Iodid zugesetzt. Anschließend wurden jeweils 100 bar Wasserstoff aufgedrückt und das flüssige Edukt bei 150°C eindosiert. Jeder Versuch wurde viermal wiederholt. Alle analytischen Messungen wurden per GC durchgeführt (Hewlett-Packard Agilent 6890, Säule CP-SIL 8). Die Produktangaben beziehen sich auf Flächenprozent im aus dem Reaktionsprodukt erhaltenen GC.

Die gemittelten Ergebnisse sind in nachstehender **Tabelle 1** zusammengefasst:

| Bsp. | Halogenid Menge [mg] | Produkte | | | | | Relative Selektivität** |
|---|---|---|---|---|---|---|---|
| | | 3,4-DCA | 2,3-DCA | CA | A | 3,4-DCNB | |
| 1* | - | 92,00 | 5,67 | 0,71 | 0,24 | 0,00 | 100,0 |
| 2 | KI [100] | 93,54 | 5,81 | 0,44 | 0,03 | 0,00 | 101,6 |
| 3 | KI [500] | 93,53 | 5,50 | 0,22 | 0,02 | 0,01 | 101,6 |
| 4 | CsI [780] | 92,93 | 5,69 | 0,27 | 0,01 | 0,00 | 101,0 |

| | | | | | | | |
|---|---|---|---|---|---|---|---|
| *: zum Vergleich. ** Die Selektivität des Vergleichsversuchs wurde mit 100 gleichgesetzt und alle anderen Selektivitäten hierauf bezogen. 3,4-DCA: 3,4-Dichloranilin; 2,3-DCA: 2,3-Dichloranilin, CA: Isomere Chloraniline; A: Anilin | | | | | | | |

## Patentansprüche

1. Verfahren zur Herstellung von Verbindungen der Formel (I) 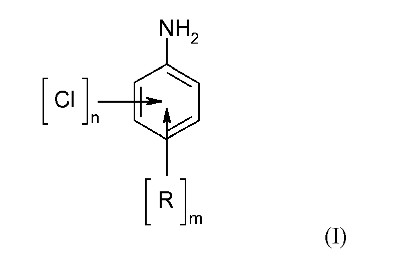 in der
n für 1, 2 oder 3 steht und
m für 0, 1, 2 oder 3 steht wobei die Summe aus m + n = 1, 2, 3, 4 oder 5 ist und R für C₁-C₆-Alkyl steht
durch Hydrierung von Verbindungen der Formel (II) 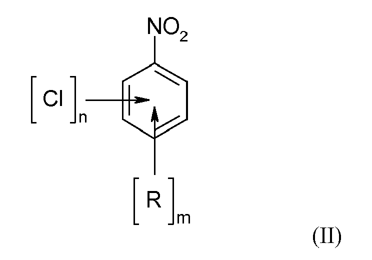 in der n, m und R die unter der Formel (I) genannte Bedeutung besitzen, in Gegenwart zumindest eines Katalysators,
das **dadurch gekennzeichnet ist, dass** der Katalysator zumindest enthält:
a) Platin und
b) Bromid oder Iodid oder Bromid und Iodid
c) Ionen zumindest eines Alkali- oder Erdalkalimetalls.

2. Verfahren nach Anspruch 1, **dadurch gekennzeichnet, dass** als Verbindungen der Formel (II) solche verwendet werden bzw. solche der Formel (I) hergestellt werden, in denen n = 2 und m = 1 oder 0 ist.

3. Verfahren nach Anspruch 1, **dadurch gekennzeichnet, dass** 2,3-Dichloranilin, 2,5-Dichloranilin, 3,4-Dichloranilin, 2,4-Dichloranilin, 3,5-Dichloranilin, 2,6-Dichloranilin oder Mischungen davon hergestellt werden.

4. Verfahren nach einem der Ansprüche 1 bis 3, **dadurch gekennzeichnet, dass** als Katalysatoren solche eingesetzt werden, die neben Platin zumindest ein weiteres Übergangsmetall enthalten, das ausgewählt ist aus der Gruppe Nickel und Kobalt.

5. Verfahren nach einem der Ansprüche 1 bis 4, **dadurch gekennzeichnet, dass** als Katalysatoren solche eingesetzt werden in denen Platin sowie die gegebenenfalls weiterhin vorhandenen Übergangsmetalle auf ein Trägermaterial aufgebracht sind.

6. Verfahren nach Anspruch 5, **dadurch gekennzeichnet, dass** der Gehalt an Platin 0,1 bis 20 Gew-% bezogen auf den Katalysator in der reduzierten und trockenen Form beträgt.

7. Verfahren nach einem der Ansprüche 1 bis 6, **dadurch gekennzeichnet, dass** der Katalysator Lithium-, Natrium-, Kalium-, Rubidium-, Cäsium-, Calcium-, Strontium oder Bariumiodid oder die Bromide der vorgenannten Alkali- und Erdalkalimetalle enthält.

8. Verfahren nach einem der Ansprüche 1 bis 7, **dadurch gekennzeichnet, dass** das molare Verhältnis von Bromid oder Iodid zur Gesamtmenge an Übergangsmetallen im Katalysator 2:1 bis 100:1 beträgt.

9. Verfahren nach einem der Ansprüche 1 bis 8, **dadurch gekennzeichnet, dass** das molare Verhältnis von Bromid oder Iodid zur Gesamtmenge an Alkalimetallionen oder zur halben Menge an Erdalkalimetallionen im Katalysator 5:1 bis 1:5 beträgt.

10. Verfahren nach einem der Ansprüche 1 bis 9, **dadurch gekennzeichnet, dass** die Reaktionstemperatur von 100 bis 250°C beträgt.

11. Verfahren nach einem der Ansprüche 1 bis 10, **dadurch gekennzeichnet, dass** es kontinuierlich durchgeführt wird.

12. Verfahren nach Anspruch 11, **dadurch gekennzeichnet, dass** die Menge des in das erfindungsgemäße Verfahren eingesetzten Katalysators bezogen auf das Gewichtsverhältnis des Katalysators in der trockenen Form zum Substrat, das pro Stunde über den Katalysator geführt wird 1:1 bis 1:5000 beträgt.

13. Katalysatoren enthaltend:
a) Platin und
b) Bromid oder Iodid oder Bromid und Iodid
c) Ionen zumindest eines Alkali- oder Erdalkalimetalls.

14. Verwendung von Katalysatoren nach Anspruch 13 zur Herstellung von Verbindungen der Formel (I) gemäß Anspruch 1.

15. Verfahren zur Herstellung von pharmazeutischen oder agrochemischen Wirkstoffen, das als Verfahrensschritt zumindest ein Verfahren nach einem der Ansprüche 1 bis 12 umfasst.
